**Europäisches Patentamt**

**European Patent Office**

**Office européen des brevets**

(19)

(11) Veröffentlichungsnummer : **0 465 969 B1**

(12) ## EUROPÄISCHE PATENTSCHRIFT

(45) Veröffentlichungstag der Patentschrift :
26.01.94 Patentblatt 94/04

(51) Int. Cl.⁵ : **B01J 23/92, B01J 27/28**

(21) Anmeldenummer : **91110830.6**

(22) Anmeldetag : **29.06.91**

(54) **Verfahren zur Reaktivierung von Ammoxidationskatalysatoren.**

(30) Priorität : **13.07.90 DE 4022416**

(43) Veröffentlichungstag der Anmeldung :
**15.01.92 Patentblatt 92/03**

(45) Bekanntmachung des Hinweises auf die
Patenterteilung :
**26.01.94 Patentblatt 94/04**

(84) Benannte Vertragsstaaten :
**DE ES GB IT NL**

(56) Entgegenhaltungen :
**EP-A- 0 165 210**
**DE-A- 2 435 031**
**DE-A- 2 717 579**

(73) Patentinhaber : **EC ERDÖLCHEMIE GMBH**
**Alte Strasse 201**
**D-50769 Köln (DE)**

(72) Erfinder : **Beuke, Brigitte**
**Alte-Neusser-Landstrasse 308**
**W-5000 Köln 71 (DE)**
Erfinder : **Herwig, Jens, Dr.**
**Nerzweg 2**
**W-5000 Köln 40 (DE)**
Erfinder : **Neeb, Ernst-Friedrich, Dr.**
**Kronenpützchen 28**
**W-4047 Dormagen 12 (DE)**
Erfinder : **Paris, Nikolaus, Dipl.-Ing.**
**von-Liebig-Strasse 4**
**W-4047 Dormagen 1 (DE)**

(74) Vertreter : **Zobel, Manfred, Dr. et al**
**BAYER AG Konzernverwaltung RP Patente**
**Konzern**
**D-51368 Leverkusen (DE)**

**Beschreibung**

Verfahren zur Reaktivierung von Ammoxidationskatalysatoren

Die Erfindung betrifft ein Verfahren zur Reaktivierung von Katalysatoren, die im Fließbettverfahren für die Ammoxidation von Olefinen eingesetzt werden. Die Reaktivierung wird durch den Zusatz eines Reaktivators durchgeführt.

Wichtige großtechnisch durchgeführte Ammoxidationsverfahren erzeugen aus Propylen oder i-Buten durch Umsetzung mit Ammoniak und Sauerstoff Acrylnitril oder Methacrylnitril. Die bisher verfügbaren und großtechnisch eingesetzten Katalysatoren zur Ammoxidation können ihre hohen Anfangsaktivitäten und besonders ihre hohen Anfangsselektivitäten nur über wenige Wochen, vielfach sogar nur über wenige Tage, halten und zeigen danach einen deutlichen Abfall, der insbesondere die gewünschte Selektivität zum (Meth)Acrylnitril betrifft, während der Gesamtumsatz des Olefins zu vermehrt auftretenden unerwünschten Beiprodukten, wie CO, $CO_2$, Acetonitril oder Blausäure im allgemeinen erhalten bleibt.

Zur Begrenzung der Ausbeuteverluste an den gewünschten Nitrilen sind verschiedene Methoden zur Reaktivierung der Ammoxidationskatalysatoren entwickelt worden. So wird in DE-A-24 35 031 ein Reaktivator eingesetzt, der aus 85 Gew.-% $MoO_3$ und 15 Gew.-% $SiO_2$ gebildet wird und der gemäß allgemeiner Beschreibung noch Fe, Bi oder Te enthalten kann und der die Ausbeute von 63 % Acrylnitril durch Zugabe von 6,4 Gew.-% Reaktivator, bezogen auf den desaktivierten Katalysator, auf 70 % Acrylnitril-Ausbeute anheben kann.

Auch im Verfahren von DE-A-33 11 521 werden hohe Gaben an Molybdänoxid eingesetzt; im angesprochenen Verfahren sind es etwa 2,2 Gew.-%, bezogen auf den desaktivierten Ammoxidationskatalysator, wobei die Acrylnitrilausbeute beispielsweise von 65,5 % auf 72,5 % steigen kann.

Eine weitere Reaktivierungsmethode ist in DE-A-27 17 579 beschrieben, wobei der desaktivierte Ammoxidationskatalysator mit einer Imprägnierlösung, die Molybdän und Wismut im Molverhältnis 0,5-20:1 und gegebenenfalls Phosphorsäure enthält, behandelt wird. Der behandelte Katalysator wird kalziniert und dann erneut eingesetzt. Im optimalen Fall wird hierbei die Selektivität für Acrylnitril von 76,7 % auf 92,8 % der Propylenumwandlung angehoben, so daß die Acrylnitril-Ausbeute bis auf 71,2 % ansteigen kann.

Eine weitere Reaktivierung wird nach dem Verfahren von DE-A-31 23 521 dadurch erreicht, daß als Reaktivator das nachkalzinierte Feinkorn eines gebrauchten Ammoxidationskatalysators im Verhältnis 1:3 zu einem desaktivierten Katalysator zugegeben wird. Hierbei können Ausbeuteverbesserungen von etwa 5 % erreicht werden.

Ganz allgemein kann natürlich auch durch quantitatives oder wenigstens teilweises Ersetzen von desaktiviertem Katalysator gegen Frischkatalysator die Acrylnitril-Ausbeute auf einem hohen Niveau gehalten werden oder von abgesunkenen Werten auf ein höheres Niveau gebracht werden. Der Nachteil dieser zuletztgenannten Methode liegt in den relativ großen Mengen, die erforderlich sind, um diesen Effekt zu erzeugen bzw. zu halten. So ist bei einem auch nur teilweisen Ersetzen eine Menge von 50 Gew.-% des ursprünglich in den Reaktor eingefüllten Katalysators pro Jahr erforderlich.

Überraschend wurde nun gefunden, daß sowohl ein desaktivierter Ammoxidationskatalysator als auch ein auf bekannte Art reaktivierter Ammoxidationskatalysator zu weiteren Ausbeuteverbesserungen befähigt werden, wenn als Reaktivator das weiter unten beschriebene Mischoxid auf einem $SiO_2$-Träger eingesetzt wird.

Es wurde ein Verfahren zur Reaktivierung von Fließbett-Katalysatoren, die für die Ammoxidation von Olefinen eingesetzt werden, durch Zusatz eines Reaktivators und unter Ausschluß von Imprägnierverfahren für die Fließbett-Katalysatoren gefunden, das dadurch gekennzeichnet ist, daß als Reaktivator ein auf $SiO_2$ als Träger angebrachtes Mischoxid der Formel

$$Mo_{20}(P,Cr)_a(Bi,Ni,Fe,Co)_bO_x \qquad (I)$$

eingesetzt wird, worin

a     einen Wert von 0,3-2 und

b einen Wert von 1,33-4 annehmen und

x     sich aus den Erfordernissen der Valenz ergibt.

Die Menge an Sauerstoff im Mischoxid und damit der Index x ergibt sich aus den Erfordernissen der Valenz. Diese Erfordernisse sind dem mit Oxidationskatalysatoren betrauten Fachmann geläufig. Hierbei wird bei dem Vorliegen der aufgeführten Elemente davon ausgegangen, daß sie in der unter den Oxidationsbedingungen höchsten stabilen Wertigkeit vorliegen.

Das Gewichtsverhältnis des Mischoxids zum $SiO_2$-Träger beträgt 0,25-1,5:1, bevorzugt 0,5-1,2:1.

Zur Herstellung des erfindungsgemäß einzusetzenden Reaktivators kann in grundsätzlich bekannter Weise der $SiO_2$-Träger mit einer oder mehreren wäßrigen Lösungen der Elementverbindungen, die zum Mischoxid führen, besprüht, getränkt oder vermischt werden. Der so erhaltene getränkte $SiO_2$-Träger wird anschließend getrocknet und danach kalziniert.

Als SiO$_2$-Träger eignet sich feines SiO$_2$ der Kornklasse <200 μm, beispielsweise 10-200 μm, bevorzugt 30-160 μm. Es ist jedoch ebenfalls möglich, daß als SiO$_2$-Träger ein SiO$_2$-Sol eingesetzt wird, und die durch Zugabe von wäßrigen Lösungen der Elementverbindungen entstehende Aufschlämmung durch Sprühtrocknung auf die gleiche Korngröße gebracht wird, die durch den Einsatz des feinteiligen SiO$_2$ der genannten Kornklasse erreicht wird.

Zur Tränkung werden wäßrige Lösungen der Elementverbindungen eingesetzt, beispielsweise solche von Ammoniumheptamolybdat, Ammoniumdihydrogenphosphat, Chrom(III)nitrat, Wismutnitrat und Nitrate der Metalle Ni, Fe und Co. Anstelle der Nitrate können grundsätzlich auch Salze anderer Anionen, beispielsweise die Acetate, eingesetzt werden, wenn solche Anionen bei der abschließenden Kalzinierung flüchtig sind und wenn sie wasserlösliche Salze ergeben. Für den Fall, daß die wäßrigen Lösungen zur Hydrolyse und zum Ausfällen von oxidischen Elementverbindungen neigen, kann durch Zusatz von wenig Salpetersäure der Zustand einer echten Lösung aufrecht erhalten werden. Der Gehalt der wäßrigen Lösungen an Elementverbindungen kann grundsätzlich in weiten Grenzen schwanken, wird aber, um die Handhabung großer Wassermengen zu vermeiden, in höheren Konzentrationsbereichen bis kurz unterhalb der Sättigungskonzentration, angewandt, beispielsweise als etwa 50 gew.-%ige wäßrige Lösung.

Die innere Oberfläche des feinteiligen SiO$_2$ beträgt im allgemeinen 100-600 m$^2$/g, bevorzugt 250-500 m$^2$/g.

Von den in Klammern gesetzten Komponentengruppen können eines oder mehrere Elemente gleichzeitig vorhanden sein.

Die Reihenfolge der Anbringung der verschiedenen Komponentengruppen für das Mischoxid auf dem SiO$_2$-Träger ist grundsätzlich beliebig. Es hat sich jedoch als vorteilhaft erwiesen, zunächst das Molybdän und die Komponentengruppe (P, Cr), in Form ihrer Verbindungen aufzutränken und danach die Komponentengruppe (Bi, Ni, Fe, Co), jeweils in Form geeigneter Verbindungen aufzutränken.

Die anschließende Trocknung kann bei 90-150°C, bevorzugt bei 110-130°C, gegebenenfalls unter vermindertem Druck erfolgen.

Im Anschluß an die Trocknung wird der imprägnierte SiO$_2$-Träger bei einer Temperatur von 600-800°C, bevorzugt 650-750°C, während einer in Abhängigkeit von der Kalzinierungstemperatur gewählten Zeit im Bereich von 5-60 min so kalziniert, daß der hierbei unter Abspaltung der obengenannten Anionen (beispielsweise Nitrat oder Acetat) erhaltene Reaktivator eine spezifische Oberfläche von 1-100 m$^2$/g, bevorzugt 5-50 m$^2$/g, besonders bevorzugt 10-30 m$^2$/g aufweist. Die Abhängigkeit der gewünschten spezifischen Oberfläche von der Kalzinierungstemperatur und der Kalzinierungsdauer ist dem Fachmann grundsätzlich bekannt und kann durch einfache Vorversuche sicher bestimmt werden.

Die zu reaktivierenden Fließbett-Katalysatoren für die Ammoxidation von Olefinen sind die bekannten und technisch eingesetzten, die z.B. in den US-Patentschriften 3 642 930 und 3 471 556 und in der DE-A-2 147 480 beschrieben sind. In besonders bevorzugter Weise wird das erfindungsgemäße Verfahren auf Ammoxidationskatalysatoren angewandt, die zur Umwandlung von Propylen in Acrylnitril eingesetzt werden.

Ein großer Vorteil des erfindungsgemäßen Verfahrens liegt in der relativ geringen spezifischen Reaktivatormenge im Bereich von 0,1-5 Gew.-%, bevorzugt 0,4-2 Gew.-%, pro Reaktivierung, bezogen auf den zu reaktivierenden Ammoxidationskatalysator. Ebenso erstaunlich ist die nachhaltige Wirkung der Reaktivierung gemäß dem erfindungsgemäßen Verfahren, so daß eine Nachdosierung/erneute Reaktivierung erst nach einigen Wochen nötig wird, während eine übliche Katalysator-Reaktivierung mit Frischkontakt eine periodische Auffrischung im Abstand von wenigen Tagen erfordert, um den gleichen Effekt zu erreichen. So ist es typisch, eine erneute Reaktivierung nach dem erfindungsgemäßen Verfahren im Abstand von einigen Wochen (etwa 4-6 Wochen) durchzuführen, so daß pro Jahr etwa 10-13, vielfach nur 10 oder 11 Reaktivierungen erforderlich sind. Selbstverständlich ist es auch möglich, in kürzeren Abständen entsprechend verkleinerte Mengen des erfindungsgemäß einzusetzenden Reaktivators zu benutzen. Der Verbrauch beträgt im Beispiel von Acrylnitril etwa 0,05-0,2 kg Reaktivator pro Tonne Acrylnitril.

Die erzielbare Erhöhung der Ausbeute an (Meth)Acrylnitril ist abhängig von der Menge an zugesetztem Reaktivator und vom Ausbeuteniveau, auf das der zu reaktivierende Ammoxidationskatalysator abgesunken war. Für den Fall, daß das Ausbeuteniveau auf einen nicht zu tiefen Wert abgesunken war, ist der Umfang der Reaktivierung geringer als bei einem auf ein niedrigeres Niveau abgesunkenen Katalysator. Typische Werte der Erhöhung der Ausbeute (Δ-Ausbeute) sind die Erhöhung von 75 auf 78 % bzw. von 72 auf 75-76 %. Die angedeuteten Zusammenhänge zwischen Ausbeuteniveau, Menge an Reaktivator und Δ-Ausbeute sind in den Ausführungsbeispielen näher erläutert.

Ein weiterer Vorteil des erfindungsgemäßen Verfahrens ist die einfache Zusammensetzung und einfache Herstellbarkeit des Reaktivators im Vergleich zu einem komplizierter aufgebauten Frischkontakt, so daß auch in diesem Punkt eine höhere Wirtschaftlichkeit der (Meth)Acrylnitril-Herstellung gesichert ist.

Beispiel A (Reaktivator-Präparation, Labormaßstab)

15,03 g SiO$_2$-Trägermaterial (Fa. Hermann) der Kornklasse <160 μm wurden vorgelegt und mit 14,96 g Ammoniumheptamolybdat (4 H$_2$O) in 15 ml Wasser und 0,325 g Ammoniumdihydrogenphosphat in 4 ml Wasser getränkt. Anschließend wurden 5,48 g Wismutnitratpentahydrat in 7 g Wasser zur weiteren Tränkung hinzugegeben.

Nach der zweiten Tränkung wurde das Gemisch bei 130°C getrocknet und anschließend bei 750°C während 15 min kalziniert, wobei eine spezifische Oberfläche von 14,7 m$^2$/g erhalten wurde.

Nach dem gleichen Präparationsschema erfolgte die Herstellung der übrigen Reaktivatoren aus der Tabelle weiter unten.

Beispiel B (Reaktivator-Präparation, großtechnisch)

18,31 kg SiO$_2$-Sol (Na-stabilisiert, 40 gew.-%ig) wurden mit 3,17 kg Ammoniumdihydrogenphosphatlösung (5 gew.-%ig), 21,44 kg Ammoniumheptamolybdatlösung (34 gew.-%ig) und 7,08 kg Wismutnitratlösung (37,7 gew.-%ig in 30 gew.-%iger Salpetersäure) gemischt. Das Gemisch wurde in einen kontinuierlich arbeitenden Sprühtrockner gegeben, um das Wasser zu entfernen und eine Kornverteilung im gewünschten Bereich von 10-150 μm bei annähernd kugelförmiger Geometrie der Teilchen zu erreichen.

Das sprühgetrocknete Material wurde in einem diskontinuierlichen Drehrohrofen bei 500°C während 20 min vom Nitrat befreit. Anschließend wurde bei 690°C während 10 min kalziniert, wobei eine spezifische Oberfläche von 15,7 m$^2$/g erhalten wurde.

Anwendungs- und Vergleichsbeispiele

allgemeine Durchführung (Laborreaktor)

In einem 1600 ml-Stahlreaktor mit einem L/D-Verhältnis von 29 wurden 550 g eines Ammoxidationskatalysators auf Molybdän-Wismutbasis mit 217 g/h Luft, 14,5 g/h Ammoniak und 30,24 g/h Propylen bei 445°C und 1,8 bar (absolut) beaufschlagt.

Das Reaktionsgas wurde analysiert und bilanztechnisch auf Umsatz, Selektivität, ACN-Ausbeute und Δ-Ausbeute (Ausbeutesteigerung) ausgewertet.

Anwendungs- und Vergleichsversuche

allgemeine Durchführung (großtechnischer Reaktor)

In einem großtechnischen Reaktor von 22,56 m Höhe und 4 m Durchmesser wurden 28,6 t eines Ammoxidationskatalysators auf Molybdän-Wismut-Basis mit 18 t/h Luft, 1,2 t/h Ammoniak und 2,57 t/h Propylen beaufschlagt. Bei einem Reaktionsdruck von 1,98 bar (absolut) wurde die Temperatur über Kühllagen, in denen Wasser verdampft, bei 445°C gehalten.

Das Reaktionsgas wurde analysiert und wie angegeben bilanztechnisch ausgewertet.

Tabelle der Beispiele 1-9 (zum Vergleich), 10-21 (Anwendung des gleichen Reaktivators auf verschiedene Katalysatoren) und 22-34 (Anwendung verschiedener Reaktivatoren).

Beispiele 1, 3 und 5-9 zeigen die katalytische Wirkung eines verschieden stark erschöpften ACN-Katalysators auf Mo-Bi-Basis; Beispiel 2 zeigt den Zusatz von 0,7 Gew.-% frischen Katalysators zum Katalysator nach Beispiel 1; Beispiel 4 zeigt die katalytische Wirkung des genannten Reaktivators für sich allein (100 %). Das Verhältnis der Komponenten ist das der Atomzahlen.

| Beispiele | Nr. | Reaktivator Menge-Gew.-% | Komponente | : Verhältnis | Umsatz % | Selekt. % | ACN-Ausb. % | Δ-Ausb. % |
|---|---|---|---|---|---|---|---|---|
| Vgl.Bsp. | 1 | - | - | : - | 99,4 | 73,1 | 75,9 | - |
| | 2 | 0,7 | Frischkat. | : Sohio C49 | 99,8 | 72,1 | 75,7 | - |
| | 3 | - | - | : - | 98,8 | 74,3 | 73,4 | - |
| | 4 | 100 | Mo,Bi,P | : 20:2,7:0,7 | 85,5 | 68,6 | 59,9 | - |
| | 5 | - | - | : - | 99,3 | 72,0 | 73,8 | - |
| | 6 | - | - | : - | 99,3 | 70,6 | 75,3 | - |
| | 7 | - | - | : - | 99,2 | 70,9 | 74,8 | - |
| | 8 | - | - | : - | 99,5 | 69,8 | 75,8 | - |
| | 9 | - | - | : - | 99,6 | 70,2 | 73,2 | - |

| Beispiele | Nr. | Basiskat. Bsp.-Nr. | Reaktivator Menge-Gew.-% | Komponente | : | Verhältnis | Umsatz % | Selekt. % | ACN-Ausb. % | Δ-Ausb. % |
|---|---|---|---|---|---|---|---|---|---|---|
| Anw.Bsp. | 10 | 1 | 0,7 | Mo,Bi,P | : | 20:2,7:0,7 | 98,7 | 74,8 | 77,8 | 1,9 |
| | 11 | 3 | 0,7 | Mo,Bi,P | : | 20:2,7:0,7 | 98,9 | 75,8 | 75,0 | 1,6 |
| | 11a | 3 | 0,7 | Mo,Bi,P | : | 20:2,7:0,7 | 98,5 | 75,6 | 74,5 | 1,1 |
| | 12 | 5 | 1,4 | Mo,Bi,P | : | 20:2,7:0,7 | 99,0 | 73,8 | 78,1 | 4,3 |
| | 13 | 6 | 0,5 | Mo,Bi,P | : | 20:2,7:0,7 | 99,3 | 73,2 | 76,8 | 1,5 |
| | 14 | 6 | 1,0 | Mo,Bi,P | : | 20:2,7:0,7 | 99,0 | 72,7 | 77,1 | 1,8 |
| | 15 | 6 | 1,5 | Mo,Bi,P | : | 20:2,7:0,7 | 98,5 | 72,8 | 76,7 | 1,4 |
| | 16 | 7 | 0,6 | Mo,Bi,P | : | 20:2,7:0,7 | 98,7 | 74,8 | 76,1 | 1,3 |
| | 17 | 8 | 1,0 | Mo,Bi,P | : | 20:2,7:0,7 | 99,4 | 73,3 | 77,4 | 1,6 |
| | 18 | 8 | 1,5 | Mo,Bi,P | : | 20:2,7:0,7 | 99,0 | 75,8 | 78,2 | 2,4 |
| | 19 | 9 | 0,4 | Mo,Bi,P | : | 20:2,7:0,7 | 99,5 | 72,3 | 75,5 | 2,3 |
| | 20 | 9 | 0,6 | Mo,Bi,P | : | 20:2,7:0,7 | 99,5 | 72,7 | 76,2 | 3,0 |
| | 21 | 9 | 0,8 | Mo,Bi,P | : | 20:2,7:0,7 | 99,5 | 73,1 | 76,2 | 3,0 |

| Beispiele Anw.Bsp. Nr. | Basiskat. Bsp.-Nr. | Reaktivator Menge-Gew.% | Komponente | : Verhältnis | Umsatz % | Selekt. % | ACN-Ausb. % | Δ-Ausb. % |
|---|---|---|---|---|---|---|---|---|
| 22 | 5 | 1,3 | Mo,Ni,P | : 20:4:0,7 | 99,1 | 72,6 | 77,8 | 4,0 |
| 23 | 5 | 1,2 | Mo,Co,P | : 20:2,7:0,7 | 99,0 | 72,3 | 77,2 | 3,4 |
| 24 | 5 | 1,2 | Mo,Fe,P | : 20:2,7:0,7 | 98,8 | 73,7 | 77,2 | 3,4 |
| 25 | 5 | 1,1 | Mo,Bi,P | : 20:1,33:0,44 | 98,8 | 71,2 | 76,5 | 2,7 |
| 26 | 5 | 1,0 | Mo,Bi,P | : 20:3,3:1,66 | 99,1 | 73,5 | 77,8 | 4,0 |
| 27 | 5 | 1,25 | Mo,Co,P | : 20:3,3:1,66 | 98,8 | 73,9 | 77,7 | 3,9 |
| 28 | 5 | 1,5 | Mo,Bi,P | : 20:4:0,7 | 98,6 | 73,5 | 77,2 | 3,4 |
| 29 | 5 | 1,2 | Mo,Co,P | : 20:4:0,7 | 99,4 | 72,5 | 77,3 | 3,5 |
| 30 | 5 | 1,0 | Mo,Fe,P | : 20:4:0,7 | 99,2 | 71,8 | 77,4 | 3,6 |
| 31 | 7 | 1,0 | Mo,Bi,Cr | : 20:2,7:0,7 | 99,2 | 74,4 | 77,7 | 3,1 |
| 32 | 7 | 1,0 | Mo,Ni,P | : 20:4:0,7 | 99,3 | 73,8 | 77,4 | 2,6 |
| 33 | 7 | 1,0 | Mo,Fe,P | : 20:4:0,7 | 99,2 | 73,6 | 77,7 | 3,1 |
| 34 | 7 | 2,0 | Mo,Co,P | : 20:4:0,7 | 99,1 | 74,2 | 76,5 | 1,7 |

## Patentansprüche

1. Verfahren zur Reaktivierung von Fließbett-Katalysatoren, die für die Ammoxidation von Olefinen eingesetzt werden, durch Zusatz eines Reaktivators und unter Ausschluß von Imprägnierverfahren für die Fließbett-Katalysatoren, dadurch gekennzeichnet, daß als Reaktivator ein auf $SiO_2$ als Träger angebrach-

7

tes Mischoxid der Formel

$$Mo_{20}(P,Cr)_a(Bi,Ni,Fe,Co)_bO_x \qquad (I)$$

eingesetzt wird, worin

a einen Wert von 0,3-2 und

b einen Wert von 1,33-4 annehmen und

x sich aus den Erfordernissen der Valenz ergibt.

2. Verfahren nach Anspruch 1, dadurch gekennzeichnet, daß das Gewichtsverhältnis des Mischoxids zum $SiO_2$-Träger 0,25-1,5:1, bevorzugt 0,5-1,2:1, beträgt.

3. Verfahren nach Anspruch 1, dadurch gekennzeichnet, daß der $SiO_2$-Träger mit einer oder mehreren wäßrigen Lösungen der Elementverbindungen, die zum Mischoxid führen, besprüht, getränkt oder vermischt wird, anschließend getrocknet wird und zum Schluß bei Temperaturen im Bereich von 600-800°C kalziniert wird.

4. Verfahren nach Anspruch 3, dadurch gekennzeichnet, daß als $SiO_2$-Träger einer der Kornklasse <200 $\mu$m, bevorzugt 10-200 $\mu$m, besonders bevorzugt 30-160 $\mu$m, eingesetzt wird.

5. Verfahren nach Anspruch 3, dadurch gekennzeichnet, daß als $SiO_2$-Träger $SiO_2$-Sol eingesetzt wird und der Reaktivator nach Anbringung der zum Mischoxid führenden Elementverbindungen durch anschließende Sprühtrocknung fertiggestellt wird.

6. Verfahren nach Anspruch 5, dadurch gekennzeichnet, daß der Reaktivator so kalziniert wird, daß er eine spezifische Oberfläche von 1-100 m²/g, bevorzugt 5-50 m²/g, besonders bevorzugt 10-30 m²/g erhält.

7. Verfahren nach Anspruch 1, dadurch gekennzeichnet, daß der Zusatz des Reaktivators 0,1-5 Gew.-%, bevorzugt 0,4-2 Gew.-%, der Menge des zu reaktivierenden Fließbettkatalysators pro Reaktivierung beträgt.

8. Verfahren nach Anspruch 1, dadurch gekennzeichnet, daß die Ammoxidation von Olefinen die des Propylens ist.

## Claims

1. Process for reactivating fluidized-bed catalysts which are used for the ammoxidation of olefins, by addition of a reactivator and exclusion of impregnation processes for the fluidized-bed catalysts, characterized in that the reactivator used is a mixed oxide of the formula

$$Mo_{20}(P,Cr)_a(Bi,Ni,Fe,Co)_bO_x \qquad (I)$$

in which

a is from 0.3 - 2,

b is from 1.33 - 4,

x is as required by the valency,

applied to $SiO_2$ as support.

2. Process according to Claim 1, characterized in that the weight ratio of the mixed oxide to the $SiO_2$ support is from 0.25 - 1.5:1, preferably from 0.5 -1.2:1.

3. Process according to Claim 1, characterized in that the $SiO_2$ support is sprayed, impregnated or mixed with one or more aqueous solutions of the compounds of the elements which lead to the mixed oxide, subsequently dried and finally calcined at temperatures in the range 600 - 800°C.

4. Process according to Claim 3, characterized in that the $SiO_2$ support used is of particle size <200 $\mu$m, preferably from 10 - 200 $\mu$m, particularly preferably from 30 - 160 $\mu$m.

5. Process according to Claim 3, characterized in that the $SiO_2$ support used is an $SiO_2$ sol and in that, after the compounds of the elements leading to the mixed oxide have been applied, the reactivator is finished by subsequent spray drying.

6. Process according to Claim 5, characterized in that the reactivator is calcined in such a way that its specific surface area is from 1 - 100 m$^2$/g, preferably from 5 - 50 m$^2$/g, particularly preferably from 10 - 30 m$^2$/g.

7. Process according to Claim 1, characterized in that the addition of reactivator per reactivation is from 0.1 - 5% by weight, preferably from 0.4 - 2% by weight, of the amount of fluidized-bed catalyst to be reactivated.

8. Process according to Claim 1, characterized in that the ammoxidation of olefins is that of propylene.

## Revendications

1. Procédé pour réactiver des catalyseurs en lit fluidisé, que l'on utilise pour l'ammoxydation d'oléfines, par addition d'un réactivateur et à l'exclusion d'un procédé d'imprégnation pour les catalyseurs en lit fluidisé, procédé caractérisé en ce que l'on utilise comme réactivateur un oxyde mixte mis sur SiO$_2$ comme support et répondant à la formule:

$$Mo_{20}(P,Cr)_a(Bi,Ni,Fe,Co)_bO_x \qquad (I)$$

dans laquelle
a est un nombre valant 0,3 à 2, et
b est un nombre valant 1,33 à 4, et
x ressort des exigences de la valence.

2. Procédé selon la revendication 1, caractérisé en ce que le rapport pondéral entre l'oxyde mixte et SiO$_2$ servant de support se situe entre 0,25 et 1,5:1, de préférence entre 0,5 et 1,2:1.

3. Procédé selon la revendication 1, caractérisé en ce que le support en SiO$_2$ est aspergé d'une ou plusieurs solutions aqueuses des composés d'éléments conduisant à l'oxyde mixte, ou bien ce support est plongé dans ou imprégné par une ou plusieurs de ces solutions ou est mélangé à une ou plusieurs de ces solutions, puis le support est séché et, à la fin, il est calciné à des températures comprises entre 600 et 800°C.

4. Procédé selon la revendication 3, caractérisé en ce que l'on utilise, comme SiO$_2$ de support une silice dont la classe de grain a moins de 200 µm, de préférence de 10 à 200 µm, de façon particulièrement préférée 30 à 160 µm.

5. Procédé selon la revendication 3, caractérisé en ce qu'on utilise comme SiO$_2$ de support un sol de SiO$_2$ et en ce que, après application des composés d'éléments conduisant à l'oxyde mixte, on achève le réactivateur par un séchage subséquent par atomisation.

6. Procédé selon la revendication 5, caractérisé en ce que le réactivateur est calciné de façon qu'il acquière une surface spécifique de 1 à 100 m$^2$/g, de préférence 5 à 50 m$^2$/g, de façon particulièrement préférée 10 à 30 m$^2$/g.

7. Procédé selon la revendication 1, caractérisé en ce que la quantité de réactivateur représente, par réactivation, 0,1 à 5 % en poids, de préférence 0,4 à 2 % en poids de la quantité du catalyseur en lit fluidisé à réactiver.

8. Procédé selon la revendication 1, caractérisé en ce que l'ammoxydation d'oléfines est celle du propylène.